# EUROPEAN PATENT SPECIFICATION

(11) **EP 4 054 485 B1**
(45) Date of publication and mention of the grant of the patent: **16.04.2025**
(21) Application number: 20811097.3
(22) Date of filing: 03.11.2020
(51) Int. Cl.: A61F 2/30, A61F 2/38

(54) **FEMORAL KNEE PROSTHESES COMPONENT AND METHOD FOR ITS CREATION**
KOMPONENTE UND VERFAHREN ZUR ERSTELLUNG VON FEMORALEN KNIEPROTHESEN
COMPOSANT ET PROCEDE DE CREATION DE PROTHESES FÉMORALES DU GENOU

(30) Priority: 08.11.2019 IT 201900020616
(43) Date of publication of application: 14.09.2022
(73) Proprietor: Permedica S.p.A., 23807 Merate (LC) (IT)
(72) Inventor: PEREGO, Marco, 23807 Merate (LC) (IT)
(74) Representative: Maccalli, Marco
(86) International application number: PCT/IB2020/060300
(87) International publication number: WO 2021/090160

(56) References cited:
- EP-A1- 1 916 007
- EP-A1- 1 923 079
- WO-A1-2019/053576
- JP-A- H09 108 249
- US-B2- 10 272 177

## Description

The present invention relates to a femoral prosthesis component for the knee joint and a method for its production.

It is known that joint prostheses are implanted in the human body in correspondence with a worn or damaged joint.

The document WO 2019053576, for example, describes a total shoulder prosthesis comprising a humeral portion and a scapular portion, each portion being provided with at least one osseointegrated component and at least one articular component. The knee joint is composed of a tibial component and a femoral component.

The femoral component comprises a pair of condyles positioned at the end of the femur whose surface slides on the corresponding surface of the tibial plateau.

The document JPH09108249 describes a knee joint prosthesis that comprises a femoral component, a tibial plateau and a tibial abutment member. The design and geometry of the articulation surfaces of the condylar members of the femoral component and the tibial abutment member is such that the contact area between the articulation surfaces is maximized during flexion of the prosthesis.

Total knee replacement surgery consists of implanting two metal joint components, one for the femur and the other for the tibia, which will form the new joint.

In order to fix the metal plates to the above-mentioned bone portions, an adhesive (cemented knee prosthesis) can be used, or the bone regrowth process (uncemented knee prosthesis) can be exploited.

When implanting a cemented knee prosthesis, the surgeon uses a fast-acting, polymethylmethacrylate-based "cement".

The disadvantage of cemented prostheses is that they are very difficult to remove, so that any possible removal and replacement intervention (due to wear, for example) is extremely difficult.

Uncemented knee prostheses have plates with a shape and materials capable of promoting bone regrowth around them.

In particular, the femoral components are characterized by a mirror-polished outer surface that reproduces the articular surface of the femur facing the tibial plateau and an internal surface in contact with the bone, appropriately surgically shaped.

Said internal surface, in the case of application without the aid of cement, is treated in such a way as to promote osseointegration, for example by means of coatings that create a porous surface that favours bone regrowth and therefore biological fixation to the femur.

It is also known that the prostheses are made of chromium-cobalt-molybdenum alloy, a material which, however, can lead to allergy phenomena.

A minimal part of these implanted metal components undergo oxidation leading to a dose-dependent release of metal ions. These can be responsible, in allergic patients, for an immune reaction with local and general consequences that can also lead, in addition to the failure of the intervention itself (with painful prostheses, reduction of joint performance, relapsing effusions) to the development of serious anaphylactic reactions.

For these reasons, particular "Nickel free" prostheses have been developed for both the hip and the knee in various materials, among which the main ones universally used are Titanium, Tivanium (TiAl₆V₄), Tantalum, Oxinium, ceramic and ceramization processed with Niobium (Niob).

Oxinium, for example, has excellent characteristics of hardness and elasticity deriving from the Zirconium surface and reduced friction, very similar, thanks to the particular processing (Zirconium oxidized with Niobium and Oxygen) to ceramic.

It is also known that the femoral components of knee prostheses are obtained by mechanical processing from a blank produced through wax casting.

The general objective of the present invention is to create a femoral component of an improved knee prosthesis to be implanted without the aid of cement (therefore with biological fixation) and which is geometrically and dimensionally identical to a traditional femoral component.

A further objective of the present invention is to provide a femoral component of a non-allergenic knee prosthesis, i.e. a prosthesis with articular surfaces which, with the same sliding and duration over time, allow there to be no release of ions ("Nickel free" prosthesis).

The objective of the present invention is also a method for producing a non-allergenic and osseointegrable prosthesis.

The above objectives are achieved by a femoral component of a knee prosthesis and a method for its creation according to the enclosed independent claims and sub-claims.

The femoral component according to the present invention is made of titanium alloy with an outer surface in contact with the polyethylene insert coated with a ceramic layer and the internal surface that can be osseointegrated with the femoral bone.

The method object of the present invention provides for the treatment of the outer surface of the femoral component through the PVD (Physical Vapour Deposition) technology and of the internal surface through the "additive manufacturing" technique in particular through the SLM technology (Selective Laser Melting).

The structural and functional characteristics of the invention and its advantages will be more clearly understandable from the following description, referring to the attached figures, which illustrate a possible non-limiting embodiment of the invention itself.

In the drawings:
- figure 1 illustrates the components of a knee prosthesis according to the known art;
- figure 2 illustrates a femoral component according to the prior art and its positioning on the femoral bone;
- figure 3 illustrates the anatomical planes of the human body;
- figures 4a and 4b illustrate the femoral component according to the present invention and the coating of the outer surface;
- figures 5a and 5b illustrate the femoral component according to the present invention and the trabecular internal surface,
- figure 6 illustrates a trabecular structure produced by means of the Electron Beam Melting technology;
- figure 7 illustrates a trabecular structure produced by means of the Selective Laser Melting technology according to the present invention.

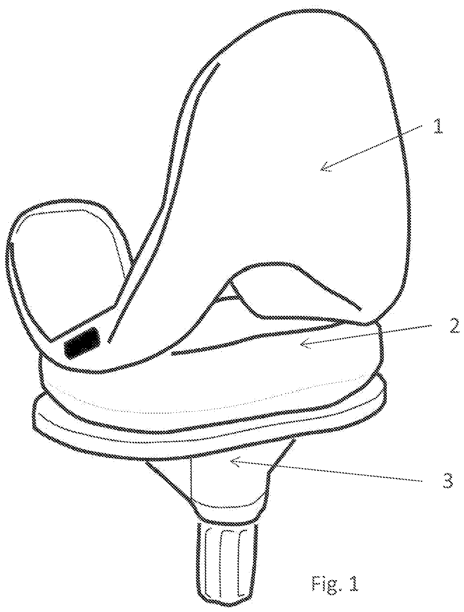

Figure 1 illustrates the components of a knee prosthesis according to the known art, which comprises a femoral component 1, a tibial component 3 and a polyethylene insert 2.

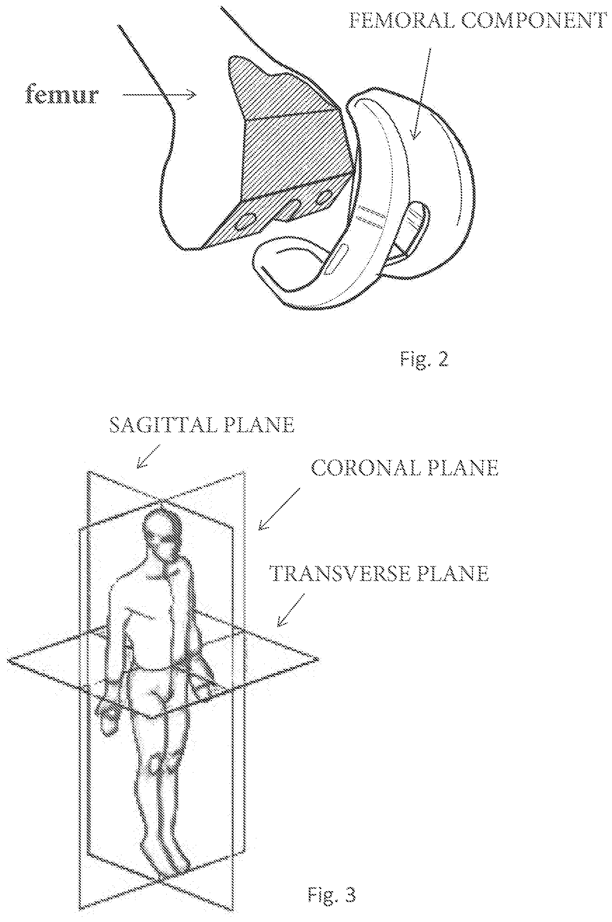

Figure 2 illustrates the positioning of the femoral component 2 on the femur.

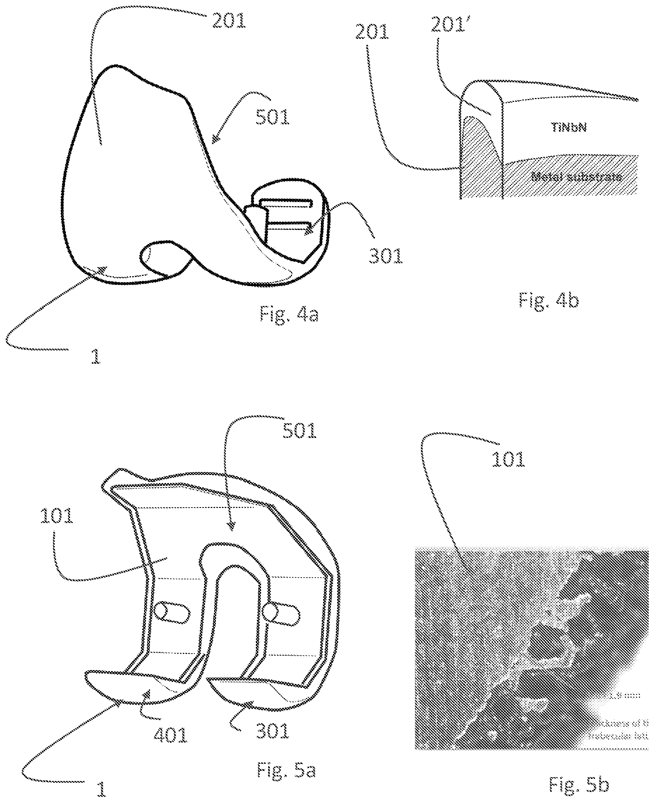

Figures 4-5b illustrate a femoral component 1 of a knee prosthesis according to the present invention.

The femoral component 1 is suitable for being implanted at the end of the femoral bone so as to be able to cooperate with a tibial component (not shown) suitable for being implanted on the tibial bone in order to reconstruct the knee joint.

The femoral component 1 has such a shape as to reproduce the condylar and patellar articulation portions of the femur.

In particular, the femoral component 1 object of the present invention has a substantially "U" shape, in cross section according to a plane parallel to the sagittal plane of the human body (figure 3), with a substantially concave internal surface 101, in contact with the femoral bone, and a substantially convex outer surface 201 suitable for being articulated with the tibial component.

Furthermore, the femoral component 1 comprises two condylar portions 301 and 401, separated by an intercondylar space, one placed laterally and one placed medially with respect to the sagittal plane of the human body, each having a similar or identical conformation to the knee condyles. Said substantially U-shaped condylar portions 301 and 401, converge and join to form a patellar portion 501, which when the prosthesis component is implanted on the femur, is in contact with the patella or in any case is positioned in the anatomical part near the patella (rotula).

According to the present invention, the femoral component 1 is made of titanium-aluminum-vanadium alloy in particular titanium 6-aluminum 4-vanadium alloy (Ti 6-AI4-V alloy) (UNI EN ISO 5832/3).

According to a further characteristic of the present invention, in order to increase the wear resistance and the tribological characteristics of the femoral component 1, the outer surface 201 is coated with at least one layer 201' of titanium and niobium nitride (TiNbN) or titanium carbonitride (TiCN).

According to a further characteristic of the present invention, in order to increase osseointegration, the internal surface 101 of the femoral component 1 is highly porous, with a trabeculation similar to that of cancellous bone, in order to allow optimal bone regrowth inside its pores and a rapid osseointegration, thus ensuring a stable anchorage of the femoral component 1 to the femoral bone.

The pores are interconnected with each other with a random irregular shape, having dimensions ranging from 100-2,000 microns.

The size of the pores is random as is also the orientation of the trabeculae in the three dimensions.

The trabecular structure is therefore characterized by a random irregular trabecular network with a high porosity and with isotropic mechanical and physical properties.

The manufacturing method of the femoral component 1 of a knee prosthesis according to the present invention provides that the internal surface 101 be produced by means of the "additive manufacturing" technique, in particular by means of the SLM (Selective Laser Melting) technology, i.e. using a laser beam as energy source, for consolidating the metal powder on the surface.

Additive manufacturing is a technology that allows a three-dimensional structure to be produced using metal material in powder form added in successive overlapping layers. In this way, a surface destined for coming into contact with the femoral bone is obtained with a highly porous trabecular structure in titanium alloy (Traser ^{®}).

Said porous surface is not a layer added to the femoral component 1 but said solid and porous internal surface 101 is produced with a continuous process in order to create a 3D metal piece.

The internal trabecular surface 101 (with irregular and interconnected open pores) mimics the porous nature of the bone, promoting bone growth and osseointegration in order to increase the stability of the implant.

The SLM (Selective Laser Melting) technology, used for producing the component according to the present invention and which provides for the fusion of titanium powder by laser, allows:
- irregular, stochastic (random) structures having an irregular shape to be created, and furthermore
- the thickness of the metal powder layer is 20-100 µm,
- the dimensions of the metal powder are 10-45 µm,
- the diameter of the laser beam is 40 µm (EOS data),
- the dimension of the melting point is 50-130 µm.

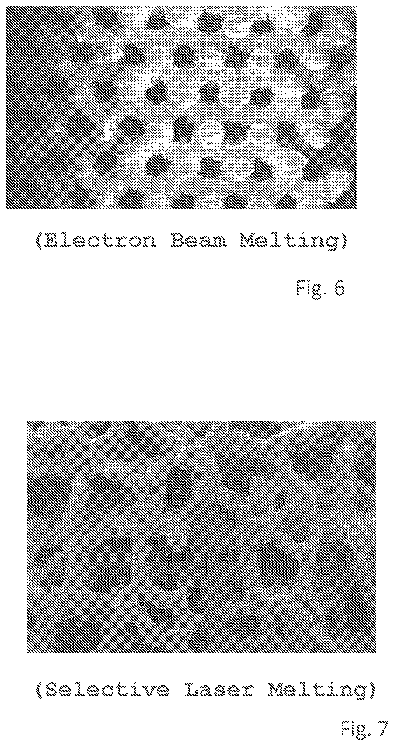

The SLM technology as can be seen in figures 6 and 7, compared to the Electron Beam Melting (EBM) technology (additive manufacturing that provides for the fusion of titanium powder by means of an electron beam to create regular hexagonal-shaped structures that mimic a diamond crystal, or in any case geometric, repeatable, developed on three planes, wherein the thickness of the metal powder layer is 50-200 µm, the dimensions of the metal powder are 45-106 µm, the diameter of the laser beam is 140 µm, Arcam Data, and the melting point dimension is 0.6 mm) has the advantage of being able to create structures with a higher level of geometric precision with lower thicknesses and more complex shapes compared to EBM. This difference in technology results in different porous metal structures. This difference can affect the regrowth and osseointegration capacity (in terms of rapidity and quantity of new tissue formed) of the bone tissue around and inside the porous structure.

Furthermore, the method for creating the femoral component 1 object of the present invention provides that the outer surface 201 alone be coated with at least one layer of titanium nitride or titanium carbonitride (TiCN) 201', which is applied by Physical Vapour Deposition (abbreviated to PVD).

The layer 201' of titanium nitride (TiNbN) or titanium carbonitride (TiCN) applied, is, for example, 4 microns.

The method provides for partially coating the femoral component 1 i.e. the outer surface 201 alone, previously cleaned, destined for coming into contact with polyethylene components of the joint.

To ensure that the coating is partial, the parts of the femoral component 1 that do not need to be coated are protected by special masks.

The method object of the present invention provides for the removable coating by means of masks or the like of the portions of the femoral component 1 which do not need to be coated with at least one layer of titanium and niobium nitride TiNbN or titanium carbonitride TiCN 201' in order to obtain a partial coating.

According to the present method, the coating of the outer surface 201 alone with at least one layer of titanium and niobium nitride (TiNbN) or titanium carbonitride (TiCN) (201') applied by physical vapour deposition (PVD) is obtained with the removable coating by means of masks or the like of the remaining surfaces of the femoral component 1 i.e. of the portions of the femoral component 1 that do not need to be coated with at least one layer of titanium nitride and niobium TiNbN or titanium carbonitride TiCN.

The TiNbN or TiCN coating is therefore a ceramic coating applied using the PVD technique (Physical Vapour Deposition for the atomic deposition of thin films, a few microns, under vacuum) on a substrate of metal material, which forms an inert and insulating layer with a barrier function between tissues and the metal of which the prosthesis is made, consequently preventing the passage of metal ions from the prosthesis to the patient.

The main function is in fact to create a physical barrier between the metal substrate and the surrounding organic environment by preventing the release of metal ions from the substrate: this significantly reduces the risk of triggering or developing allergic reactions caused by the metal alloy elements.

Furthermore, thanks to its high degree of hardness and abrasion resistance, it also improves the tribological performance of the articular surfaces of the metal alloy of which the femoral component 1 is made, thus decreasing the wear of the polyethylene of the insert 2.

The objectives of the invention mentioned in the preamble of the description have thus been achieved.

The protection scope of the present invention is defined by the enclosed claims.

## Claims

1. A femoral component (1) of an osseointegrable knee prosthesis at one end of the femur, having a substantially U- shaped form, in cross section according to a plane parallel to the sagittal plane of the human body, with a substantially concave internal surface (101), in contact with the femoral bone, and a substantially convex outer surface (201) suitable for articulating with a tibial component of said prosthesis,
**characterized in that**:
said femoral component (1) is in titanium alloy 6-aluminum 4-vanadium (Ti 6-AI4-V) (UNI EN ISO 5832/3);
the outer surface (201) of said femoral component (1) is coated with at least one layer (201') of titanium and niobium nitride (TiNbN) or titanium carbonitride (TiCN);
the internal surface (101) of said femoral component (1) has a trabecular structure, said trabecular structure having an irregular random network of trabeculae with a high porosity and with isotropic mechanical and physical properties.

2. A method for the creation of a femoral prosthesis component (1) for the osseointegrable knee joint at one end of the femur in correspondence with the knee j oint according to claim 1, **characterized in that** the internal trabecular surface (101) is formed using the "additive manufacturing" technique with the SLM (Selective Laser Melting) technology and the outer surface (201) alone is coated with at least one layer of titanium and niobium nitride (TiNbN) or titanium carbonitride (TiCN) (201') applied by physical vapor deposition (PVD).

3. The method according to claim 2, **characterized in that** the coating of the outer surface (201) alone with at least one layer of titanium and niobium nitride (TiNbN) or titanium carbonitride (TiCN) (201') applied by Physical Vapour Deposition (PVD) is obtained with the removable coating by means of masks or the like of the remaining surfaces of the femoral component (1), i.e. of the portions of the femoral component (1) which do not have to be coated with at least one layer of titanium and niobium nitride (TiNbN) or titanium carbonitride (TiCN).

## Patentansprüche

1. Eine Femurkomponente (1) einer Osseointegrationsknieprothese an einem Ende des Femurs, die eine im wesentlichen U-förmige Form aufweist, im Querschnitt gemäß einer Ebene parallel zur Sagittalebene des menschlichen Körpers, mit einer im wesentlichen konkaven Innenfläche (101), die in Kontakt mit dem Femurknochen steht, und einer im wesentlichen konvexen Außenfläche (201), die geeignet ist, mit einer Tibiakomponente der Prothese zu artikulieren,
**dadurch gekennzeichnet, dass**:
die Femurkomponente (1) aus einer 6-Aluminium-4-Vanadium (Ti 6-Al4-V) (UNI EN ISO 5832/3) Titanlegierung besteht;
die Außenfläche (201) der Femurkomponente (1) mit mindestens einer Schicht (201') aus Titan- und Niobnitrid (TiNbN) oder Titancarbonitrid (TiCN) beschichtet ist;
die Innenfläche (101) der Femurkomponente (1) eine trabekuläre Struktur aufweist, wobei die trabekuläre Struktur ein unregelmäßiges, zufälliges Netz von Trabekeln mit einer hohen Porosität und mit isotropen mechanischen und physikalischen Eigenschaften aufweist.

2. Verfahren zur Herstellung einer Femurprothesenkomponente (1) für das Osseointegationskniegelenk an einem Ende des Femurs in Übereinstimmung mit dem Kniegelenk nach Anspruch 1, **dadurch gekennzeichnet, dass** die innere trabekuläre Oberfläche (101) durch die Technik der "additiven Herstellung" mit der SLM-Technologie (Selective Laser Melting) gebildet wird und die äußere Oberfläche (201) allein mit mindestens einer Schicht aus Titan- und Niobnitrid (TiNbN) oder Titancarbonitrid (TiCN) (201') beschichtet wird, die durch physikalische Dampfabscheidung (PVD) aufgebracht wird.

3. Verfahren nach Anspruch 2, **dadurch gekennzeichnet, dass** die Beschichtung der Außenfläche (201) allein mit mindestens einer Schicht aus Titan- und Niobnitrid (TiNbN) oder Titancarbonitrid (TiCN) (201'), die durch physikalische Dampfabscheidung (PVD) aufgebracht wird, mit der entfernbaren Beschichtung mittels Masken oder dergleichen der übrigen Oberflächen der Femurkomponente (1), d. h. der Teile der Femurkomponente (1), die nicht mit mindestens einer Schicht aus Titan- und Niobnitrid (TiNbN) oder Titancarbonitrid (TiCN) beschichtet werden müssen, erreicht wird.

## Revendications

1. Composant fémoral (1) d'une prothèse de genou ostéointégrable à une extrémité du fémur, ayant une forme sensiblement en U, en section transversale selon un plan parallèle au plan sagittal du corps humain, avec une surface interne sensiblement concave (101), en contact avec l'os fémoral, et une surface externe sensiblement convexe (201) adapté pour s'articuler avec un composant tibial de ladite prothèse,
**caractérisé en ce que** :
ledit composant fémoral (1) est en alliage de titane 6-aluminium 4-vanadium (Ti 6-Al4-V) (UNI EN ISO 5832/3) ;
la surface externe (201) dudit composant fémoral (1) est revêtue d'au moins une couche (201') de nitrure de titane et de niobium (TiNbN) ou de carbonitrure de titane (TiCN),
la surface interne (101) dudit composant fémoral (1) a une structure trabéculaire, ladite structure trabéculaire a un réseau aléatoire irrégulier de travées à porosité élevée et à propriétés mécaniques et physiques isotropes.

2. Procédé de création d'un composant de prothèse fémorale (1) pour l'articulation du genou ostéointégrable à une extrémité du fémur en correspondance avec l'articulation du genou selon la revendication 1, **caractérisé en ce que** la surface trabéculaire interne (101) est formée en utilisant la technique de « fabrication additive » avec la technologie SLM (fusion laser sélective) et la surface extérieure (201) seule est revêtue d'au moins une couche de nitrure de titane et de niobium (TiNbN) ou de carbonitrure de titane (TiCN) (201') appliquée par dépôt physique en phase vapeur (PVD).

3. Procédé selon la revendication 2, **caractérisé en ce que** le revêtement de la surface extérieure (201) seule avec au moins une couche de nitrure de titane et de niobium (TiNbN) ou de carbonitrure de titane (TiCN) (201') appliquée par dépôt physique en phase vapeur (PVD) est obtenu avec le revêtement amovible au moyen de masques ou analogues des surfaces restantes du composant fémoral (1), c'est-à-dire des parties du composant fémoral (1) qui ne doivent pas être revêtues d'au moins une couche de nitrure de titane et de niobium (TiNbN) ou de carbonitrure de titane (TiCN) .
